# EUROPEAN PATENT APPLICATION

(11) **EP 1 172 142 A1**
(43) Date of publication of application: **16.01.2002**
(21) Application number: 00909371.7
(22) Date of filing: 13.03.2000
(51) Int. Cl.: B01J 29/89, C01B 39/48, C07D 301/12, C07D 301/19

(54) **MCM-41 TYPE MICROPOROUS MATERIALS CONTAINING TITANIUM AND THEIR UTILIZATION AS CATALYSTS IN ALPHA-PINENE OXIDATION**

(30) Priority: 16.03.1999 ES 9900565
(71) Applicant: CONSEJO SUPERIOR DE INVESTIGACIONES CIENTIFICAS, 28006 Madrid (ES); UNIVERSIDAD POLITECNICA DE VALENCIA, 46022 Valencia (ES)
(72) Inventor: CORMA CANOS, Avelino, Instituto de Tec. Quimica, Naranjos, 46022 Valencia (ES); DOMINE, Marcelo E., Instituto de Tec. Quimica, Naranjos, 46022 Valencia (ES); SUSARTE ROGEL, Manuel, Instituto de Tec. Quimica, Naranjos, 46022 Valencia (ES); REY GARCIA, Fernando, Instituto de Tec. Quimica, Naranjos, 46022 Valencia (ES)
(74) Representative: Ungria Lopez, Javier
(86) International application number: ES0000087
(87) International publication number: WO0054880

(57) **Abstract**

**OXIDATION OF α-PINENE**

The present invention describes the use of MCM-41 type mesoporous solid catalysts based on silica that also contain Ti in their composition in order to obtain the products from the oxidation of α-pinene using organic hydroperoxides as oxidizing agents.

## Description

### TECHNICAL FIELD

Heterogeneous catalysis

### BACKGROUND

The production of epoxides derivatives of terpenes is of interest in the field of the synthesis of fragrances and food additives (K. Bauer et al. *"Common Fragrance and Flavor Materials: Preparation, Properties and Use",* 2^{nd} Ed. VCH Publishers, NY, **1985).** Generally, this process is carried out industrially using per-acids (W.K. Anderson et al. *J. Org. Chem.* 38, **1973,** 2267), organic hydroperoxides (H. Wulff et al. *US Pat* 3,642,833; 3,923,843; 4,021,545; 4,367,342; D.V. Banthorpe et al. *Chem. Ind*., 14, **1981,** 502), hydrogen peroxide (C. Venturello et al. *J. Org. Chem.,* 53, **1988,** 1553), iodous-compounds (M.F. Texeira et al. *Catal. Lett.,* 38, **1996,** 133 and 42, **1996,** 213,) or sodium hypochlorite carrying out the oxidation reaction in a homogeneous phase or in two-phase systems (R.A. Sheldon et al. *Catal. Today,* 19, **1994,** 215; CATTECH, Dec. **1997,** 95). The use of homogeneous catalysts based on transaction metals such as W, Mo, Ru, Ti, Mn, Fe and Co among others, has also been described (P. Barret et al. *Pharm. Acta Helv.,* 62, **1987,** 348; N. Fdil et al. *J. Mol. Catal.,* 108, **1996,** 15; M. Lajunm et al. *Tet. Lett,* 35, **1994,** 4461; D.D. Aregawal et al. *Indian J. Chem., Sect. A,* 36A(2), **1997** 164). Nonetheless, none of the described cases have obtained improvements applicable to the industrial scale.

The developments of catalysts based on microporous titanosilicates with a zeolitic structure (M. Taramasso et al. *US Pat.* 4,410,501, **1983)** opened a new field in oxidation processes permitting oxidation of linear olefins (C. Neri et al. *EP Pat* 100,119, **1984;** F. Maspero et al. *EP Pat.* 190,609, **1986:** M.G. Clerici et al. *EP Pat*. 230,949, **1987)** and hydroxylation of phenol with hydrogen peroxide (A. Esposito et al. *US Pat.* 2,116,974, **1985;** Ad*vances in Catalysis,* 41, **1996,** 253). However, these materials due to their small pore opening, prevent their use in processes in which larger molecules are involved such as is the case of terpenes. In this sense, the synthesis of MCM-41 type mesoporouos materials containing Ti in their composition made their use possible in oxidation reactions of voluminous molecules (A. Corma et al. *SP Pat.* 9,301,327, **1993** extended to PCT/ES94/00059, **1994.**

This invention claims the use of titanosilicates as MCM-41 type mesoporous catalysts in the reaction of oxidation of α-pinene with organic hydroperoxides, giving rise to the corresponding epoxide and campholenic aldehyde.

### DESCRIPTION OF THE INVENTION

This invention claims the use of MCM-41 type mesoporous catalysts based on silica containing Ti in their composition that may or may not have organic groups anchored to their surface in selective oxidation processes of α-pinene with organic hydroperoxides such as terbutylhydroperoxide or cumene hydroperoxide, without these examples being restrictive.

This reaction is carried out by contacting a reactive mixture that contains α-pinene and the organic hydroperoxide with the MCM-41 type mesoporouos solid catalyst at a temperature between 10 and 200°C with constant stirring during reaction times that may vary between 5 minutes and 24 hours depending on the catalyst and on the reaction conditions used. The hydrophilicity-hydrophobicity properties of the catalyst may be modified by anchoring organosiliceous compounds to the surface of the MCM-41 type mesoporous solid and adapt them to the specific characteristics of the reagents. The inclusion of titanium in the MCM-41 type mesoporous catalyst may be done by means of direct synthesis, wherein a titanium precursor is added to the synthesis gel, or else by means of anchoring titanium compounds to a silica surface that gives rise to isolated Ti species after a calcination process. Finally, in the event that structure directing organic compounds are used during the synthesis of the MCM-41 type mesoporous material, the elimination of the organic species that are found inside the pores of the MCM-41 type mesoporous material can be carried out by means of calcination of the catalyst or by means of chemical extraction thereof.

In this way highly active and selective catalysts in oxidation processes of α-pinene have been obtained.

The present invention describes a process by means of which products from the oxidation of α-pinene, basically epoxides and aldehydes, with high conversions and selectivities, are obtained. This method is based on the use of MCM-41 type mesoporous solid catalysts based on silica that contain titanium in their composition.

The catalyst has the following molar composition:

SiO₂ : x TiO₂ : n S

where x may vary between 0.0001 and 0.5, S may be an organic compound, such as for example a cationic, anionic or neutral surfactant. The cationic surfactants respond to the formula R₁R₂R₃R₄Q⁺ wherein Q is nitrogen or phosphorous and wherein at least one of the substituents R₁, R₂, R₃ or R₄ is an aryl or alkyl group containing more than 6 carbon atoms and less than 36, and each one of the remaining groups R₁, R₂, R₃ or R₄ is hydrogen or an alkyl or aryl group with less than five carbons. The so-called geminal surfactants, R₁R₂R₃QR₄QR₁R₂R₃ or R₁R₂R₃Q(R₄R₅QR₆QR₄R₅)QₙR₁R₂R₃ wherein Q is nitrogen or phosphorous and at least one of the substitutents R₁-R₆ is an alkyl or aryl group with more than six carbon atoms and less than 36, and each one of the remaining groups R₁-R₆ are hydrogen or alkyl or aryl groups with less than five carbon atoms or mixtures thereof, may also be included in the cationic surfactants that may be included in the composition of the gel. In these cases two of the groups R₁, R₂, R₃ or R₄ may be interconnected giving rise to cycled compounds. The cationic surfactants are introduced in the composition of the synthesis gel in the form of hydroxide, halide, nitrate, sulfate, carbonate or silicate or mixtures thereof. Non-restrictive examples thereof are cetyltrimethylammonium, dodecyltrimethylammonium, cetylpyridinium, cetyltrimetylphosphonium, etc.

S may also refer to a neutral surfactant, in which case the formula is R₁R₂R₃Q wherein Q is nitrogen or phosphorous and wherein at least one of the substitutents R₁, R₂ or R₃ is an aryl or alkyl group containing more than 6 carbon atoms and less than 36, and each one of the remaining groups R₁, R₂ or R₃ is hydrogen or an alkyl or aryl group with less than five carbons, dodecylamine, cetylamine and cetylpriridine being non-restrictive examples. Compounds that respond to the formula nR-EO that consist of an alkylpolyethylene oxide, alkyl-arylpolyethylene oxides and alkylpolypropylene and alkylethylene copolymers may also act as neutral surfactants. Commercial surfactants named Tergitol 15-S-9, Triton X-114, Igepal RC-760, Pluronic 64 L, Tetronic and Sorbitan may be non-restrictive examples thereof.

Esters derived from fatty acids obtained by reaction with short chain alcohols, sugars, amino acids, amines and polymers or copolymers derived from polypropylene, polyethylene, polyacrylamide or polyvinylalcohol may also be included in the formulation. Non-restrictive examples are lysolecithin, lecithin, pentaoxyethylene ether dodecyl, phosphatyldilauryldiethanolamine, digalactose diglyceride and monogalactose diglyceride. The surfactant may also be an anionic surfactant that responds to the formula RQ⁻ wherein R is an aryl or alkyl group containing more than 6 carbon atoms and less than 36, and Q is a sulfate, carboxylic, phosphate or sulfate group, non-restrictive examples being dodecylsulfate, stearic acid, Aerosol OT and phospholipids such as phosphatyl choline and diethanolamine phosphatyl and n may vary between 0 and 0.5.

The synthesis of these MCM-41 type mesoporous catalysts is carried out by preparing a gel with a molar composition:

SiO₂ : x TiO₂ : n S : m TAAOH

wherein x may have values between 0.0001 and 0.5 and S may be a cationic, anionic or neutral surfactant. The cationic surfactants respond to the formula R₁R₂R₃R₄Q wherein Q is nitrogen or phosphorous and wherein at least one of the substituents R₁, R₂, R₃ or R₄ is an aryl or alkyl group containing more than 6 carbon atoms and less than 36, and each one of the remaining groups R₁, R₂, R₃ or R₄ is hydrogen or an alkyl or aryl group with less than five carbons. The so-called geminal surfactants R₁R₂R₃QR₄QR₁R₂R₃ or R₁R₂R₃Q(R₄R₅QR₆QR₄R₅)ₙQR₁R₂R₃ wherein Q is nitrogen or phosphorous and at least one of the substituents R₁-R₆ is an alkyl or aryl group with more than six carbon atoms and less than 36, and each one of the remaining groups R₁-R₆ are hydrogens or alkyl or aryl groups with less than five carbon atoms or mixtures thereof, may also be included in the cationic surfactants that may be included in the gel composition. In these cases two of the groups R₁, R₂, R₃ or R₄ may be interconnected giving rise to cycled compounds. The cationic surfactants are introduced in the composition of the synthesis gel in the form of hydroxide, halide, nitrate, sulfate, carbonate or silicate or mixtures thereof. Non-restrictive examples are cetyltrimethylammonium, dodecyltrimethylammonium, cetylpyridinium, cetyltrimethylphosphonium, etc.

S may also refer to a neutral surfactant, in which case the formula is R₁R₂R₃Q wherein Q is nitrogen or phosphorous and wherein at least one of the substituents R₁, R₂ or R₃ is an aryl or alkyl group containing more than 6 carbon atoms and less than 36, and each one of the remaining groups R₁, R₂ or R₃ is hydrogen or an alkyl or aryl group with less than five carbons, non-restrictive examples being dedecylamine, cetylamine and cetylpyridine. Compounds that respond to the formula nR-EO that consist of alkylpolyethylene oxides, alkylarylpolyethylene oxides and alkylpolypropylene and alkylethylene copolymers may also act as neutral surfactants. The commercial surfactants named Tergitol 15 S 9, Triton X-114, Igepal RC-760, Pluronic 64 L, Tetronic and Sorbitan are non-restrictive examples thereof. Esters derived from fatty acids obtained by reaction with short chain alcohols, sugars, amino acids, amines and polymers or copolymers derived from polypropylene, polyethylene, polyacrylamide or polyvinylalcohol may also be included in the formula. Non-restrictive examples are lysolecithin, lecithin, pentaoxyethylene dodecyl ether, dilauryldiethanolamine phosphatyl, digalactose diglyceride and monogalactose diglyceride. The surfactant may also be an anionic surfactant that responds to the formula RQ⁻ wherein R is an aryl or alkyl group containing more than 6 carbon atoms and less than 36, and Q is a sulfate, carboxylic, phosphate or sulfate group, non-restrictive examples being dodecylsulfate, stearic acid, Aerosol OT and phospholipids such as phosphatyl choline and diethanolamine phosphatyl, n may vary between 0 and 5. TAAOH refers to a tetralakylammonium hydroxide, tetraarylammonium or arylalkylammonium, ammonium, alkali metal, alkaline earth metal or mixtures thereof, m may vary between 0 and 10.

The synthesis of these materials is carried out by preparing an aqueous, alcohol solution or water/alcohol mixture containing TAAOH. A pure or dissolved silicon source is added to the preceding solution with constant stirring and at temperatures between 0 and 90°C. Finally, a pure or dissolved titanium source is added to the reaction mixture. Oxides, oxyhydroxides, alkoxides, halides or any of the salts thereof may be used as Ti and/or Si sources, and in general any Ti and/or Si compound capable of being hydrolyzed in the reaction conditions may be used. This solution also contains the surfactant.

The resulting mixture is stirred until complete homogeneity for times between 0.1 minutes and 60 hours for the purpose of eliminating part or all of the alcohols that may have been introduced in the synthesis gel.

The resulting mixture is introduced in an autoclave and heated between 20 and 200°C for a time between 10 minutes and 60 hours. The final solids are separated from the mother liquors, washed with water, alcohol or water-alcohol mixtures and dried.

The organic part occluded in the pores of the materials may be eliminated by means of calcination at temperatures between 300 and 1100°C, or else by treatment with a mixture of one or several mineral organic acids in a solvent that may be water, alcohol, hydrocarbons or mixtures thereof. Sulfuric acid, nitric acid, hydrochloric acid, perchloric acid, acetic acid, mono-, di- or trichloroacetic acid, mono-, di- or trifluoroacetic acid are preferred as acids, these examples being non-restrictive. The purpose of this treatment is to extract the surfactant or other organic residue that may be occluded inside the pores of the catalyst. This treatment is carried out at temperatures between 0 and 250°C in one or more successive extraction steps, although two or three steps are generally sufficient so as to extract all of the organic part from the inside of the pores. The duration of this treatment is comprised between 10 minutes and 40 hours depending on the acid or mixture of acids used, the extraction temperature, solvent and liquid/solid ratio, the range preferred for the latter being between 5 and 100 g.g⁻¹.

These materials have a high specific surface comprised between 200 and 1,500 m².g⁻¹ and have an intense band in the UV-vis spectrum centered around 220 nm, which indicates the presence of Ti in tetrahedral environments. These catalysts are active and selective in reactions of olefin epoxydation in general, and reactions of α-pinene epoxydation in particular.

These materials may also be prepared by means of anchoring Ti species to the hydroxylated surface of a purely siliceous MCM-41 type mesoporous material. In this case, the silicic precursor is prepared in a way similar to the one explained above, but it does not include any reagent that includes titanium in its preparation. The purely silicic MCM-41 type mesoporouos solid is dehydrated and contacted with a titanium compound in gas phase or in organic solution, a catalyst that favors the reaction between the Si-OH groups of the MCM-41 type mesoporouos material and the titanium compound being eventually present, the catalyst being an amine, ammonia or an organic or inorganic hydroxide. The preferred titanium compounds are: titanium halides, titanium alkoxides, dichlorotitanocene or titanium complexes wherein the titanium atom is coordinated by a dionate group such as acetylacetonate, ammonium or sodium hexafluorotitanate and any complex or ionic salt that contains titanium in its composition and that may be capable of reacting with a Si-OH group in the conditions in which the claimed material has been prepared. The excess reagents are eliminated by washing and/or heat treatment in an inert atmosphere, followed by calcination in air in conditions such that the organic matter or halogens that may be present in the material are eliminated.

These materials have a high specific surface comprised between 200 and 1,500 m².g⁻¹ and have an intense band in the UV-vis spectrum centered around 220 nm, which indicates the presence of Ti in tetrahedral environments. These catalysts are active and selective in reactions of olefin epoxydation in general, and reactions of α-pinene epoxydation in particular.

In an additional step the material may be treated with a methylating agent. This methylation is carried out using R₁R₂R₃(R')Y, R₁R₂(R')₂Y, R₁(R')₃Y or R₁R₂R₃Y-NH-Y R₁R₂R₃ wherein R₁, R₂ and R₃ are organic groups that are the same or are different from each other and they may be H or alkyl or aryl groups that may or may not be functionalized with amines, thiols, sulfonic, groups, tetralkylammoniums or acids, R' is a group hydrolizable in the conditions of preparation such as for example alkoxide or halide groups. Y is a metal among which Si, Ge, Sn or Ti is preferred. Methylation processes are well known in the art. In this way most of the Si-OH and Ti-OH groups present in the original material is functionalized.

The chemical composition of the resulting methylated material is defined as:

SiO₂ : *y* YR_{*p*}O₂₋_{*p*}_{/2} : *x* TiO₂ : *a* H₂O

wherein R is hydrogen or an alkyl, aryl or polyaromatic group, the same or different from each other, that may or may not be functionalized with acid, amino, thiol groups, etc. and that is bonded directly to the atoms that comprise the structure by means of C-Y bonds, Y may be Si, Ge, Sn or Ti, *p* may vary between 1 and 3, *y* may have values between 0.0001 and 1, *x* may vary between 0.00001 and 0.25, preferably between 0.0001 and 0.1 and a depends on the degree of hydration of the material and may vary between 0 and 2.

The methylated materials have a high specific surface comprised between 100 and 1,500 m².g⁻¹ and have an intense band in the UV-vis spectrum centered around 220 nm, which indicates the presence of Ti in tetrahedral environments. These catalysts are active and selective in reactions of olefin epoxydation in general and of α-pinene epoxydation in particular.

The following examples illustrate the preparation of these materials and the application thereof to the reaction of selective oxidation of α-pinene.

### EXAMPLES

### Example 1: Preparation of a MCM-41 type mesoporous material containing Ti in its composition.

3.11 g of cetryltrimethylammonium bromide (CTAB) are dissolved in 20.88 g of water. 5.39 g of tetramethylammonium hydroxide (TMAOH) and 0.21 g of titanium tetraethoxide (TEOT) are added to this solution and stirred until the titanium compound has completely dissolved. Then, 3.43 g of silica are added giving rise to a gel that is stirred at room temperature for 1 hour at 250 r.p.m. The resulting mixture is introduced in autoclaves and heated to 100°C at the autogenous pressure of the system for 48 hours. After this period of time has gone by, a solid is recovered by filtration, washed thoroughly with distilled water and dried at 60°C for 12 hours.

### Example 2: Activation of a catalyst like the one described in example 1 by calcination.

3.00 g of the material described in example 1 are placed in a quartz tubular reactor and a stream of dry nitrogen of 50 ml.min⁻¹ is passed through while the temperature is raised up to 540°C at 3°C.min⁻¹. Once this temperature has been reached nitrogen is passed through for 60 minutes, after which, the flow of nitrogen is replaced by a flow of dry air of 50 ml.min⁻¹. Calcination continues for 360 minutes more and the solid is cooled at room temperature. This heat treatment permits all the organic part occluded in the pores of the material to be eliminated.

This solid has a specific surface of 950 m².g⁻¹, as well as a band in which the UV-vis spectrum is centered at 220 nm.

### Example 3: Use of a material like the one described in example 2 as a selective catalyst in the reaction of oxidation of α-pinene.

150 g of a material like the one described in example 2 are introduced in a glass reactor at 70°C that contains 1,140 mg of α-pinene and 1,890 of cumene hydroperoxide. The reaction mixture is stirred and a sample is taken after 8 hours of reaction. The conversion of α-pinene is 10.00% and the yields to epoxide and campholenic aldehyde are 2.07% and 4.42%, respectively. The efficiency of the oxidizing agent is 27.54%.

### Example 4: Methylation of a material like the one described in example 2.

2.0 g of the sample obtained in example 2 are dehydrated at 100°C and 10⁻³ Tor for 2 hours. The sample is cooled, and a solution of 1.88 g of hexamethyldisilazane (CH₃)₃Si-NH-Si(CH₃)₃) in 30 g of toluene is added at room temperature. The resulting mixture is refluxed at 120°C for 90 minutes and washed with toluene. The final product is dried at 60°C.

This solid has a specific surface of 935 m².g⁻¹, as well as a band in the UV-vis spectrum centered at 220 nm. Furthermore, the ²⁹Si-MAS-NMR spectrum has a resonance band at -10 ppm assigned to the presence of Si-C bonds.

### Example 5: Use of a material like the one described in example 4 as a selective catalyst in the reaction of oxidation of α-pinene.

150 mg of a material like the one described in example 4 are introduced in a glass reactor at 70°C that contains 1,140 mg of α-pinene and 1,890 of cumene hydroperoxide. The reaction mixture is stirred and a sample is taken after 8 hours of reaction. The conversion of α-pinene is 40.37% and the yields to epoxide and campholenic aldehyde are 33.38% and 2.43%, respectively. The efficiency of the oxidizing agent is 92.88%.

### Example 6: Activation of a material like the one described in example 1 by chemical extraction.

5.5 g of a sample like the one described in example 1 are treated with 276.4 g of a solution of 0.05 M sulfuric acid in ethanol. This suspension is stirred under reflux for one hour. The solid is recovered by filtration and washed with ethanol to a neutral pH. The resulting solid is dried at 100°C for 30 minutes, obtaining 3.51 g of the product. The resulting solid is subjected to a second extraction step in which 3.5 g of solid are added to an 0.15 M solution of hydrochloric acid in ethanol/heptane (48:52), using a liquid/solid ratio of 50. This suspension is refluxed with constant stirring for 24 hours, filtered and washed with ethanol. The resulting solid is dried at 60°C for 12 hours.

This solid has a specific surface of 983 m².g⁻¹, as well as a band in the UV-vis spectrum centered at 220 nm.

### Example 7: Use of a material like the one described in example 6 as a selective catalyst in the reaction of oxidation of α-pinene.

150 mg of a material like the one described in example 6 are introduced in a glass reactor at 70°C that contains 1,140 mg of α-pinene and 1,890 of cumene hydroperoxide. The reaction mixture is stirred and a sample is taken after 8 hours of reaction. The conversion of α-pinene is 38.06% and the yields to epoxide and campholenic aldehyde are 26.48% and 4.92%, respectively. The efficiency of the oxidizing agent is 56.61%.

### Example 8: Methylation of a material like the one described in example 7.

2.0 g of the sample obtained in example 7 are dehydrated at 100°C and 10⁻³ Tor for 2 hours. The sample is cooled and a solution of 1.88 g of hexamethyldisilazane (CH₃)₃Si-NH-Si(CH₃)₃) in 30 g of toluene is added at room temperature. The resulting mixture is refluxed at 120°C for 90 minutes and washed with toluene. The final product is dried at 60°C.

This solid has a specific surface of 965 m².g⁻¹, as well as a band in the UV-vis spectrum centered at 220 nm. Furthermore, the ²⁹Si-MAS-NMR spectrum has a resonance band at -10 ppm assigned to the presence of Si-C bonds.

### Example 9: Use of a material like the one described in example 8 as a selective catalyst in the reaction of oxidation of α-pinene.

150 mg of a material like the one described in example 8 are introduced in a glass reactor at 70°C that contains 1,140 mg of α-pinene and 1,890 of cumen hydroperoxide. The reaction mixture is stirred and two samples are taken after half an hour and 8 hours of reaction. The conversion of α-pinene after half an hour of reaction is 49.17% and the yields to epoxide and campholenic aldehyde are 40.66% and 3.51%, respectively. The efficiency of the oxidizing agent is 70.96% and the conversion of α-pinene after eight hours of reaction is 60.58% and the yields to epoxide and campholenic aldehyde are 30.95% and 14.6%, respectively. The efficiency of the oxidizing agent is 96.62%.

### Example 10: Preparation of a MCM-41 type mesoporous material to which titanium is introduced in post-synthesis treatment.

31.10 g of cetyltrimethylammonium bromide (CTAB) are dissolved in 20.88 g of water. 53.90 g of tetramethylammonium hydroxide (TMAOH) are added to this solution and stirred until complete homogeneity. Then 34.30 g of silica are added giving rise to a gel that is stirred at room temperature for 1 hour at 250 rpm. The resulting mixture is introduced in autoclaves and heated to 100°C at the autogenous pressure of the system for 48 hours. Afterwards, a solid is recovered by filtration, thorough washing with distilled water and dried at 60°C for 12 hours.

10.00 g of the material obtained are placed in a quartz tubular reactor and a stream of dry nitrogen of 50 ml.min⁻¹ is passed through while the temperature is raised to 540°C at 3C.min⁻¹. Once said temperature has been reached nitrogen is passed through for 60 minutes, after which, the nitrogen flow is replaced by a dry air flow of 50 ml.min⁻¹. Calcination continues for 360 minutes and the solid is cooled to room temperature. This heat treatment allows the complete elimination of all the organic part occluded in the pores of the material.

This solid has a specific surface of 976 m².g⁻¹.

The inclusion of titanium is carried out by anchoring a titanium compound to the surface of the purely siliceous sample.

5 g of the material are dehydrated at 300°C and 10⁻³ mm of Hg for 2 hours, adding a solution that contains 0.079 g of titanocene dichloride in 45 g of anhydrous chloroform. The resulting suspension is stirred at room temperature for 1 hour under an Ar atmosphere. A solution that contains 0.063 g of triethylamine in 10 g of chloroform is added to this suspension. White gases are given off and the color of the solution changes from orangey-yellow to orangey-red. Stirring continues for one hour. The solid is recovered by filtration and the excess reagents are eliminated by thorough washing with dichloromethane. The resulting solid is calcined at 540°C in a N₂ atmosphere for 1 hour and the heat treatment continues for 6 more hours in air. Under these conditions all the organic part present in the material is eliminated.

The MCM-41 type mesoporous material containing Ti does not show any significant differences with respect to the purely siliceous precursor.

The UV-vis spectrum of this material has a narrow band at 220 nm assigned to the formation of monomeric titanium species.

### Example 11: Use of a material like the one described in example 10 as a selective catalyst in the reaction of oxidation of α-pinene.

150 mg of a material like the one described in example 10 are introduced in a glass reactor at 70°C that contains 1,140 mg of α-pinene and 1,890 of cumene hydroperoxide. The reaction mixture is stirred and a sample is taken after 8 hours of reaction. The conversion of α-pinene is 52.50% and the yields to epoxide and campholenic aldehyde are 32.10% and 8.00% respectively. The efficiency of the oxidizing agent is 55.00%.

### Example 12: Methylation of a material like the one described in example 10.

2.0 g of the sample obtained in example 10 are dehydrated at 100°C and 10⁻³ Tor for 2 hours. The sample is cooled and a solution of 1.88 g of hexamethyldisilazane (CH₃)₃Si-NH-Si(CH₃)₃) in 30 g of toluene is added at room temperature. The reaction mixture is refluxed at 120°C for 90 minutes and washed with toluene. The final product is dried at 60°C.

This solid has a specific surface of 930 m².g⁻¹, as well as a band in the UV-vis spectrum centered at 220 nm. Furthermore, the ²⁹Si-MAS-NMR spectrum has a resonance band at -10 ppm assigned to the presence of Si-C bonds.

### Example 13: Use of a material like the one de scribed in example 12 as a selective catalyst in the reaction of oxidation of α-pinene.

150 mg of a material like the one described in example 12 are introduced in a glass reactor at 70°C that contains 1,140 mg of α-pinene and 1,890 of cumene hydroperoxide. The reaction mixture is stirred and a sample is taken after 8 hours of reaction. The conversion of α-pinene is 53.70% and the yields to epoxide and campholenic aldehyde are 35.80% and 7.10%, respectively. The efficiency of the oxidizing agent is 69.20%.

## Claims

1. Use of MCM-41 type mesoporouos materials formed by Si and Ti in the form of titanosilicates and that may also contain organic compounds bonded to the Si and/or Ti atoms that form the structure as catalysts for the oxidation of α-pinene.

2. Use of MCM-41 type mesoporous materials as catalysts for the oxidation of α-pinene that respond to the chemical formula:
SiO₂ : x TiO₂ . n S
wherein x may vary between 0.0001 and 0.5, S may be an organic compound, such as for example a cationic, anionic or neutral surfactant. The cationic surfactants respond to the formula R₁R₂R₃R₄Q⁺ wherein Q is nitrogen or phosphorous and wherein at least one of the substituents R₁, R₂, R₃ or R₄ is an aryl or alkyl group containing more than 6 carbon atoms and less than 36, and each one of the remaining groups R₁, R₂, R₃ or R₄ is hydrogen or an alkyl or aryl group with less than five carbons. The so-called geminal surfactants R₁R₂R₃QR₄QR₁R₂R₃ or R₁R₂R₃Q(R₄R₅QR₆QR₄R₅)QₙR₁R₂R₃ wherein Q is nitrogen or phosphorous and at least one of the substitutents R₁-R₆ is an alkyl or aryl group with more than six carbon atoms and less than 36, and each one of the remaining groups R₁-R₆ are hydrogen or alkyl or aryl groups with less than five carbon atoms or mixtures thereof, are also included in the cationic surfactants that may be included in the composition of the gel. In these cases two of the groups R₁, R₂, R₃ or R₄ may be interconnected giving rise to cycled compounds. The cationic surfactants are introduced in the composition of the synthesis gel in the form of hydroxide, halide, nitrate, sulfate, carbonate or silicate or mixtures thereof. Non-restrictive examples thereof are cetyltrimethylammonium, dodecyltrimethylammonium, cetylpyridinium, cetyltrimethylphosphonium, etc.
S may also refer to a neutral surfactant, in which case it responds to the formula R₁R₂R₃Q wherein Q is nitrogen or phosphorous and wherein at least one of the substituents R₁, R₂ or R₃ is an aryl or alkyl group containing more than 6 carbon atoms and less than 36 and each one of the remaining groups R₁, R₂ or R₃ is hydrogen or an alkyl or aryl group with less than five carbons, non-restrictive examples being dodecylamine, cetylamine and cetylpiridine. Compounds that respond to the formula nR-EO that consist of alkylpolyethylene oxides, alkylarylpolyethylene oxides and alkylpolypropylene and alkylethylene copolymers, may also act as neutral surfactants. The commercial surfactants named Tergitol 15-S-9, Triton X-114, Igepal RC-760, Pluronic 64 L, Tetronic and Sorbintan are non-restrictive examples thereof. The formulation may also include esters derived from fatty acids obtained by reaction with short chain alcohols, sugars, amino acids, amines and polymers or copolymers derived from polypropylene, polyethylene, polyacrylamide or polyvinylalcohol, non-restrictive examples being lysolechithin, lecithin, pentaoxiethylene dodecyl ether, phosphatyllauryldiethanolamine, digalactose diglyceride and monogalactose diglyceride. The surfactant may also be an anionic surfactant that responds to the formula RQ⁻ wherein R is an aryl or alkyl group containing more than 6 carbon atoms and less than 36, and Q is a sulfate, carboxylic, phosphate or sulfate group, non-restrictive examples being dodecylsulfate, stearic acid, Aerosol OT and phospholipids such as phosphatyl choline and diethanolamine phosphatyl and n may vary between 0 and 0.5, wherein the organic material corresponding to the group S is extracted chemically.

3. The use of MCM-41 type mesoporous materials as catalysts for the oxidation of α-pinene that are prepared like the ones described in claim 2 but wherein they are subjected in a subsequent step to a silanization process giving rise to the formation of species that contain Si-C bonds.

4. The use of MCM-41 type mesoporous materials as catalysts for the oxidation of α-pinene that are prepared like the ones described in claim 2, but that differ therefrom in that the groups containing Si-C bonds are introduced during a synthesis step prior to the elimination of the organic part.

5. The use of MCM-41 type mesoporous materials as catalysts for the oxidation of α-pinene that are prepared like the ones described in claim 2 but that differ therefrom in that the organic part corresponding to the group S is eliminated by means of a calcination step.

6. The use of MCM-41 type mesoporous materials as catalysts for the oxidation of α-pinene that are prepared like the ones described in claim 5 but wherein they are subjected to a silanization process in a subsequent step giving rise to the formation of species that contain Si-C bonds.

7. The use of MCM-41 type mesoporous materials as catalysts for the oxidation of α-pinene that are prepared like the ones described in claim 2 wherein the organic part corresponding to the S group is eliminated by means of an extraction process by means of treatment with a solution of a mineral or organic acid in a solvent that may be water, alcohol, hydrocarbons or mixtures thereof.

8. The use of MCM-41 type mesoporous materials as catalysts for the oxidation of α-pinene that are prepared like the ones described in claims 3 and 6 wherein compounds that respond to the formula R₁R₂R₃(R')Y, R₁R₂(R')₂Y, R₁(R')₃Y or R₁R₂R₃Y-NH-Y R₁R₂R₃, wherein R₁, R₂ and R₃ are the same or are different from each other and may be H or alkyl or aryl groups that may or may not be functionalized with amines, thiols, sulfonic groups, tetraalkylammoniums or acids are used as silanizing agents, R' is a group hydrolizable in the conditions of preparation such as for example alkoxide or halide groups. Y is a metal among which Si, Ge, Sn or Ti is preferred, the methylation processes being well known in the art. Most of the Si-OH and Ti-OH groups present in the MCM-41 type mesoporous materials are functionalized in this way.

9. Use of MCM-41 type mesoporous materials as supports of the active phases of titanium resulting in active catalysts for the oxidation of α-pinene that respond to the chemical formula:
SiO₂ : n S
wherein x may vary between 0.0001 and 0.5, S may be an organic compound, such as for example a cationic, anionic or neutral surfactant. The cationic surfactants respond to the formula R₁R₂R₃R₄Q⁺ wherein Q is nitrogen or phosphorous and wherein at least one of the substituents R₁, R₂, R₃ or R₄ is an aryl or alkyl group containing more than 6 carbon atoms and less than 36, and each one of the remaining groups R₁, R₂, R₃ or R₄ is hydrogen or an alkyl or aryl group with less than five carbons. The so-called geminal surfactants R₁R₂R₃QR₄QR₁R₂R₃ or R₁R₂R₃Q(R₄R₅QR₆QR₄R₅)QₙR₁R₂R₃ wherein Q is nitrogen or phosphorous and at least one of the substitutents R-R₆ is an alkyl or aryl group with more than six carbon atoms and less than 36, and each one of the remaining groups R₁-R₆ are hydrogen or alkyl or aryl groups with less than five carbon atoms or mixtures thereof, are also included in the cationic surfactants that may be included in the composition of the gel. In these cases two of the groups R₁, R₂, R₃ or R₄ may be interconnected giving rise to cycled compounds. The cationic surfactants are introduced in the composition of the synthesis gel in the form of hydroxide, halide, nitrate, sulfate, carbonate or silicate or mixtures thereof. Non-restrictive examples thereof are cetyltrimethylammonium, dodecyltrimethylammonium, cetylpyridinium, cetyltrimethylphosphonium, etc.
S may also refer to a neutral surfactant, in which case it responds to the formula R₁R₂R₃Q wherein Q is nitrogen or phosphorous and wherein at least one of the substituents R₁, R₂ or R₃ is an aryl or alkyl group containing more than 6 carbon atoms and less than 36 and each one of the remaining groups R₁, R₂ or R₃ is hydrogen or an alkyl or aryl group with less than five carbons, non-restrictive examples being dodecylamine, cetylamine and cetylpiridine. Compounds that respond to the formula nR-EO that consist of alkylpolyethylene oxides, alkylarylpolyethylene oxides and alkylpolypropylene and alkylethylene copolymers, may also act as neutral surfactants. The commercial surfactants named Tergitol 15-S-9, Triton X-114, Igepal RC-760, Pluronic 64 L, Tetronic and Sorbintan are non-restrictive examples thereof. The formulation may also include esters derived from fatty acids obtained by reaction with short chain alcohols, sugars, amino acids, amines and polymers or copolymers derived from polyrpolyene, poliethylene, polyacrylamide or polyvinylalcohol, non-restrictive examples being lysolechithin, lecithin, pentaoxiethylene dodecyl ether, phosphatyllauryldiethanolamine, digalactose diglyceride and monogalactose diglyceride. The surfactant may also be an anionic surfactant that responds to the formula RQ⁻ wherein R is an aryl or alkyl group containing more than 6 carbon atoms and less than 36, and Q is a sulfate, carboxylic, phosphate or sulfate group, non-restrictive examples being dodecylsulfate, stearic acid, Aerosol OT and phospholipids such as phosphatyl choline and diethanolamine phosphatyl and n may vary between 0 and 0.5.
The organic material corresponding to the group S is extracted chemically.

10. Use of MCM-41 type mesoporous materials as catalysts for the oxidation of α-pinene that respond to the chemical formula:
SiO₂ : x TiO₂
that is obtained by supporting active titanium species on MCM-41 type mesoporous supports like the ones described in claim 9 and wherein x may vary between 0.0001 and 0.5. Titanium compounds among which titanium halides, titanium alkoxides, dichlorotitanocene or titanium complexes in which the titanium atom is coordinated by a dionate group such as acetylacetonate, ammonium or sodium hexafluorotitanate and any complex or ionic salt that contains titanium in its composition and that may be susceptible of reacting with a Si-OH group, are preferred, are used a precursors of the active titanium species. The excess reagents are eliminated by washing and/or heat treatment in an inert atmosphere, followed by calcination in air under such conditions that the organic matter or halogens that might be present in the final MCM-41 type mesoporous titanosilicate material is eliminated.

11. The use of MCM-41 type mesoporous materials as catalysts for the oxidation of α-pinene that are prepared like the ones described in claim 10 but wherein they are subjected to a silanization process in a subsequent step giving rise to the formation of species that contain Si-C bonds.

12. The use of MCM-41 type mesoporous materials as catalysts for the oxidation of α-pinene that are prepared like the ones described in claim 11 wherein compounds that respond to the formula R₁R₂R₃(R')Y, R₁R₂(R')₂Y, R₁(R')₃Y or R₁R₂R₃Y-NH-Y R₁R₂R₃, wherein R₁, R₂ and R₃ are the same or are different from each other and may be H or alkyl or aryl groups that may or may not be functionalized with amines, thiols, sulfonic groups, tetraalkylammoniums or acids are used as silanizing agents, R' is a group hydrolizable in the conditions of preparation such as for example alkoxide or halide groups. Y is a metal among which Si, Ge, Sn or Ti is preferred, the methylation processes being well known in the art. Most of the Si-OH and Ti-OH groups present in the MCm-41 type mesoporous materials are functionalized in this way.

13. The use of MCM-41 type mesoporous materials as catalysts for the oxidation of α-pinene that are prepared like the ones described in claims 2 to 8 and 10 to 12 and wherein an organic hydroperoxide such as for example, without these being non-restrictive examples, cumene hydroperoxide, terbutylhydroperoxide and ethyl-phenylhydroperoxide, is used as an oxidizing agent.

14. A process of oxidation of α-pinene to the corresponding epoxide or campholenic aldehyde that is characterized in carrying out in a batch reactor, a continuous stirring type reactor or continuous fixed bed reactor using a MCM-41 type mesoporous solid that contains silicon and titanium in its composition, and optionally a group that contains Si-C or Ti-C bonds, and prepared according to claims 1 to 12, and organic hydroperoxides such as for example, without these being non-restrictive examples, terbutylhydroperoxide, cumene hydroperoxide or ethylbenzene hydroperoxide, as oxidizing agents.

15. A process of oxidation of α-pinene to the corresponding epoxide or campholenic aldehyde according to claim 14 wherein the reaction temperature is between 25 and 350°C.

16. A process of oxidation of α-pinene to the corresponding epoxide or campholenic aldehyde according to claims 14 and 15 wherein the reaction times are between 2 minutes and 24 hours.

17. A process of oxidation of α-pinene to the corresponding epoxide or campholenic aldehyde according to claims 14, 15 and 16 wherein the reactor is a batch type one and the weight ratio of α-pinene to catalyst is between 1 to 10, and the molar ratio α-pinene/organic hydroperoxide is between 7 and 0.5.

18. A process of oxidation of α-pinene to the corresponding epoxide or campholenic aldehyde according to claims 14, 15, 16 and 17 wherein the reaction products are separated by distillation and the non-reacted α-pinene is recycled to the reactor.
